# EUROPEAN PATENT APPLICATION

(11) **EP 4 738 369 A1**
(43) Date of publication of application: **06.05.2026**
(21) Application number: 25207943.9
(22) Date of filing: 10.10.2025
(51) Int. Cl.: G16B 25/10, G16B 40/20, C12Q 1/6886

(54) **A METHOD OF ANALYZING A SAMPLE FOR OVARIAN CANCER DETECTION USING A MACHINE LEARNING MODEL**

(30) Priority: 31.10.2024 PL 45019224
(71) Applicant: Gdanski Uniwersytet Medyczny, 80-210 Gdansk (PL)
(72) Inventor: JOPEK, Maksym, 84-106 Wielka Piasnica (PL); SUPERNAT, Anna, 81-586 Gdynia (PL); SIECZCZYNSKI, Michal, 13-200 Dzialdowo (PL); PASTUSZAK, Krzysztof, 82-300 Elblag (PL); ZACZEK, Anna, 81-575 Gdynia (PL)
(74) Representative: Godlewski, Piotr

(57) **Abstract**

The subject of the invention is a method for analyzing a biological material sample for the detection of ovarian cancer, comprising isolating RNA from platelets, preparing cDNA libraries, fragmenting, molecularly indexing with Illumina barcodes, sequencing, and bioinformatic processing, in which the cDNA libraries, after bioinformatic processing, are mapped to the human reference genome (hg19), then samples with a read count greater than 100,000 per sample are selected, the data is normalized using a variance-stabilizing transformation, model training is performed on training data and classification performance is assessed using a validation set; in the final stage, the sample is evaluated by the resulting algorithm, which classifies the sample as "ovarian cancer" or "healthy".

## Description

The subject of the invention is a method for analyzing a biological material sample: preferably RNA derived from blood platelets for the early detection of ovarian cancer (OC) using a trained machine-learning model. OC poses a major challenge in oncology due to its asymptomatic nature in the early stages, which leads to late diagnosis and a five-year survival rate of only 50%. The lack of effective screening tests for asymptomatic, average-risk patients further hampers early-stage detection, underscoring the urgent need for innovative methods to identify this malignancy. Although the serum biomarker CA-125 is routinely used in clinics, it is not suitable as a screening tool due to its low sensitivity in early OC and a high rate of false positives (elevated CA-125 levels may also accompany menstruation, pregnancy, or endometriosis).

According to the state of the art, liquid biopsies most commonly use cell-free circulating DNA (cfDNA) or circulating tumor cells (CTCs). In case of cfDNA, success in detecting ovarian cancer was reported by J. D. Cohen et al., 2018, and by D. Killock, 2018, where the CancerSEEK diagnostic test was developed to simultaneously assess protein biomarker concentrations and cfDNA (containing a fraction of circulating tumor DNA). CancerSEEK achieved a high OC detection rate of around 98%. However, for samples from stage I patients, this test demonstrated only 40% sensitivity. Using the same dataset, K. C. Wong et al., 2019, obtained approximately 96% sensitivity for OC detection, and ~77% and ~90% for stages I and II, respectively, at 99% specificity when detecting any type of cancer using a semi-naive Bayesian machine-learning model (CancerA1DE). In the publication by S. Rahaman, X. Li, J. Yu, and K.-C. Wong et al., 2019, the authors achieved 99% sensitivity at 99% specificity in cancer detection using a one-dependence estimators ensemble model (CancerEMC). The first study employed 39 biomarkers and considered patient sex, whereas the second used only 15 biomarkers combined with age, ethnicity, and sex. Another multi-cancer screening test, Galleri (GRAIL), disclosed in C. Sheridan, 2017, achieved a 67% detection rate for OC at 99% specificity in the early stage. However, the clinical utility of this test proved limited due to high costs.

In light of these studies and their limitations, the use of platelet RNA represents a valuable alternative for OC detection and more. Changes in the platelet RNA profile are detectable at early stages, offering a chance to capture the disease even during its asymptomatic phase.

In M. A. Jopek et al., 2024, it was shown that applying logistic regression to platelet RNA expression data enables 76.5% sensitivity at 99% specificity for OC detection (72% sensitivity at a 99% specificity threshold for early detection). The ImPlatelet tool disclosed in K. Pastuszak et al., 2021, based on deep learning, is another example of the potential of platelet-based diagnostics, achieving up to 88% specificity with 95% sensitivity. Furthermore, as presented by Y. Gao et al., 2022, combining platelet RNA with the CA-125 marker yielded 85.3% specificity at 86.4% sensitivity in samples from various OC stages and 73.2% sensitivity at 86.0% specificity in case of early OC detection.

It should be noted here that, while cross-study comparisons are difficult to perform due to differing metrics and their levels, they nonetheless show the trade-offs between sensitivity and specificity and the importance of a balanced diagnostic approach. In addition, the many studies also differ by applying various methods for normalizing test samples, notably: separate normalization of the test and training sets, and joint normalization of test and training data.

The objective of the invention is to address the lack of tools for early ovarian cancer diagnostics using blood as a liquid-biopsy material, enabling a minimally invasive alternative to traditional methods. An additional objective is to improve diagnostic methodology by developing specialized classifiers tailored to different demographic cohorts.

Currently, the biggest problem for detecting ovarian cancer is low sensitivity of tests. Our developed solution is characterized by high sensitivity for detecting stages I and II for a model intended for screening, and high specificity for a high-risk group.

The subject of the invention is a method for analyzing a biological material sample for early detection of ovarian cancer, comprising isolating RNA from platelets, preparing cDNA libraries, fragmenting, molecular indexing with Illumina barcodes, sequencing, and bioinformatic processing, characterized in that the cDNA libraries, after bioinformatic processing, are mapped to the human reference genome (hg19), then a cDNA sample with a read count greater than 100,000 per sample is selected, the data are normalized using a variance-stabilizing transformation, and the model is trained on training-subset based on assessment of classification quality using a validation set, after which the sample is evaluated by the resulting algorithm, which classifies the sample as "ovarian cancer" or "healthy".

Preferably, the method is characterized in that each cDNA sample contains information on RNA expression levels for 5,277 genes measured in platelets.

Preferably, the method is characterized in that the algorithm was trained on samples from two independent cohorts 1 and 2, which were combined and normalized using the DESeq2 package in R.

Preferably, the method is characterized in that the samples from the cohorts for training dataset originate from healthy donors and donors with OC.

Preferably, the method is characterized in that the training of the algorithm was limited to samples from asymptomatic healthy women and women with OC.

Preferably, the method is characterized in that, for all cancer stages, the specificity of the method is at least 54.9% for a model with 100% sensitivity and the sensitivity is at least 74.5% for a model with 100% specificity when only women are included in the model.

Preferably, the method is characterized in that, for all cancer stages, the specificity of the method is at least 35.2% for a model with 100% sensitivity and the sensitivity is at least 52.9% for a model with 100% specificity when only OC are included in the model.

Preferably, the method is characterized in that, for all cancer stages, the specificity of the method is at least 40.8% for a model with 100% sensitivity and the sensitivity is at least 53.9% for a model with 100% specificity when only women and OC are included in the model.

Preferably, the method is characterized in that, for all cancer stages, the specificity of the method is at least 59.2% for a model with 100% sensitivity and the sensitivity is at least 75.5% for a model with 100% specificity when the platelet RNA profile and donor age are included in the model as a separate feature.

The solution according to the invention enables earlier diagnosis of ovarian cancer using a machine-learning model based on liquid-biopsy data (platelet RNA), trained with additional clinical information and with partitioning of the training datasets that considers donor sex and tumor location. In addition, the effect of including donor age as a separate feature was examined when creating diagnostic models as an additional variable.

The method according to the invention includes construction of classifiers optimized for: (a) broader population screening with an emphasis on high specificity; and (b) monitoring of a high-risk group with an emphasis on high sensitivity.

Commercial application of such a test would enable large-scale OC screening for women. When drawing blood for routine tests, the patient could be offered to extend the diagnostics with the said test. In the future, the test could be expanded to include additional diagnostic targets based on patients' RNA profiles, such as detection of cancers other than OC. The developed solution has the potential to facilitate rapid intervention and improve treatment outcomes.

The novelty of the method according to the invention lies in the use of clinical data in combination with the platelet RNA expression profile for OC diagnostics using a trained and optimized Logistic Regression model. Unlike similar available methods that are often applied to the diagnosis of all cancer types, the method according to the invention focuses on ovarian cancer and on defining the training set in such a way as to maximize early-stage sample classification sensitivity while maintaining 100% specificity. In addition to patient platelet RNA data, patient age was included as an additional variable during model training. Furthermore, an individualized approach was applied to the analysis of new samples, consisting of normalizing new data sample by sample together with the training-validation set after prior model training, thereby eliminating any simultaneous access to test data during the algorithm-training process. This approach enables faster cancer detection, which is often decisive for the patient's health. In a broader perspective, it will lead to better treatment outcomes.

The early-stage (I and II) OC diagnostic model created according to the invention achieves sensitivity of 75.8% at 100% specificity for data from the training-validation cohort (cohort 1), and 81.3% balanced accuracy for samples from an independent dataset (cohort 3) for the screening model. In the case of the model aimed at the high-risk group, the focus was on detecting cancer at any stage of development, resulting in 60.6% specificity at 100% sensitivity for data from cohort 1, and 50% balanced accuracy for data from cohort 3. Compared to existing OC detection methods, this method offers superiority in detecting ovarian cancer at an early stage.

An advantage of the method according to the invention is that it provides a solution combining clinical data with the platelet RNA expression profile in OC diagnostics, because:
a) it uses donor age as a separate feature;
b) it enables verification of how dataset selection affects prediction;
c) it employs a trained logistic regression model for screening purposes and for the high-risk group, focusing, respectively, on high specificity and sensitivity of the developed test; and
d) unlike earlier studies that used standard normalization techniques, it introduces an individualized approach to each newly analyzed sample by normalizing new data sample by sample together with the training-validation set after prior model training.

Such a detailed analysis, encompassing clinical data and an in-depth model analysis, has not previously been conducted. The described method focuses exclusively on ovarian cancer and early-stage disease detection. This approach enables faster cancer detection, which translates into better treatment outcomes.

### Figure description

The subject of the invention is explained in exemplary embodiments and in the drawings, in which:

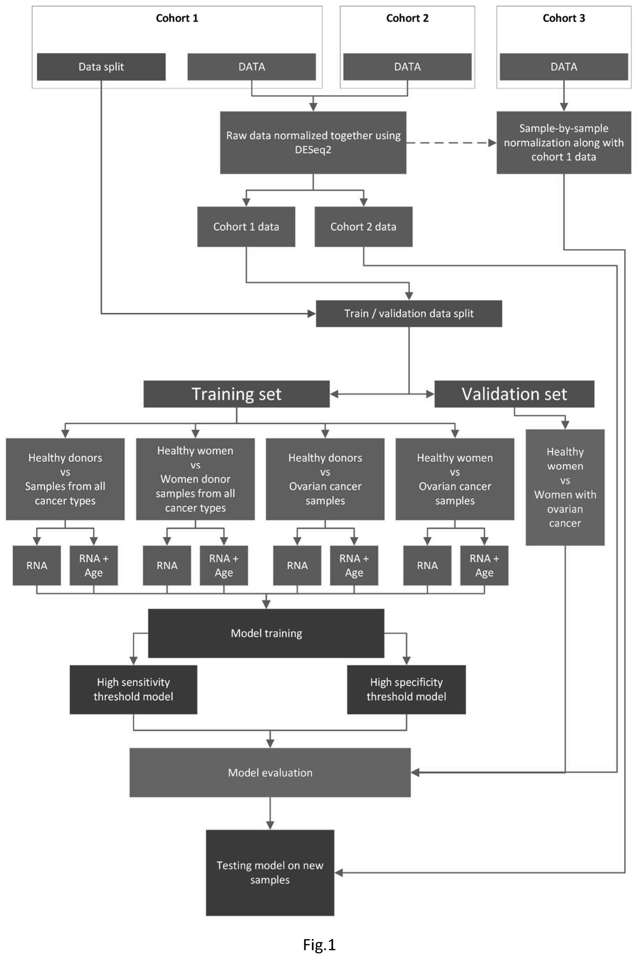

Fig. 1 shows a description of the experimental workflow leading to a reliable result of "ovarian cancer" or "healthy" based on a blood sample.

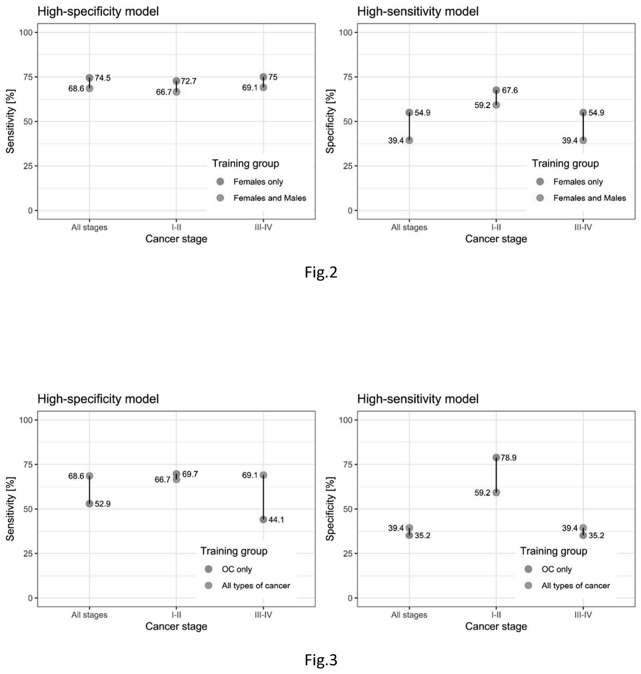

Fig. 2 shows the results of classification based on a logistic regression model, where the model was trained in two ways: with and without including samples from men. The results indicate that training the model on women only yields higher test sensitivity and specificity. Sensitivity and specificity results of the developed test are presented by disease stage.
Fig. 3 shows the results of classification based on a logistic regression model, where the model was trained in two ways: on all available data and on data originating only from patients with OC. The results indicate that training the model on all cancer types increases the late-stage classification, whereas training only on samples representing ovarian cancer increases the early stage classification. Sensitivity and specificity results of the developed test are presented by disease stage.

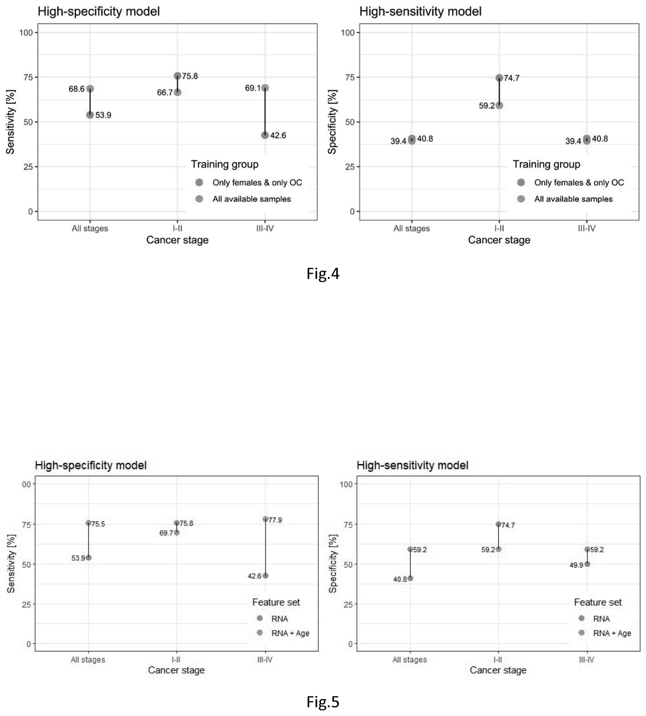

Fig. 4 shows the results of classification based on a logistic regression model, where the model was trained in two ways: on all available data and on data originating only from healthy women and patients with OC. The results indicate that training the model exclusively on healthy women and women with ovarian cancer does not increase sensitivity in case of late-stage detection, but improves the early stage classification in High Sensitivity model, and in High Specificity model. Sensitivity and specificity results of the developed test are presented by disease stage.
Fig. 5 shows the results of classification based on the most precise model for late stage cancer classification, with the additional inclusion of donor age as a separate feature. Both the sensitivity and specificity of the test are higher in advanced disease stages when age is analyzed alongside the platelet RNA profile. Sensitivity and specificity results of the developed test are presented by disease stage.

### Examples of embodiments

The examined material comprises blood platelets collected from oncology patients and healthy controls, also referred to as asymptomatic donors, i.e., individuals who, at the time of blood collection, did not have cancer, nor any other diagnosed disease. It is recommended to begin processing the samples as soon as possible after collection-within 30 minutes to 48 hours-to preserve RNA integrity. Platelet isolation is performed by centrifugation to obtain platelet-rich plasma, after which the platelets are pelleted and resuspended in an appropriate buffer (e.g., RNAlater). Alternative isolation methods may be used, such as filtration or magnetic depletion of leukocytes using anti-CD45 antibodies, to increase the purity of the isolated platelets. RNA is then isolated using commercial kits (mirVana miRNA Isolation Kit, DirectZol) or by classical phenol-chloroform extraction. The quality and quantity of the isolated RNA are assessed with devices such as the Bioanalyzer or TapeStation using RNA Pico chips or similar analytical tools. For cDNA synthesis, kits such as the SMARTer Ultra Low RNA Kit for Illumina Sequencing or the KAPA Stranded mRNA-Seq Kit may be used. The cDNA is then fragmented. The prepared libraries are coupled with molecular barcodes using kits such as the TruSeq Nano DNA Sample Prep Kit. The libraries are evaluated for quality and quantity, and high-quality samples are pooled and prepared for sequencing. Sequencing is performed on Illumina platforms such as HiSeq 2000, HiSeq 2500, or HiSeq 4000, in single-end mode with read lengths of 50 or 100 base pairs, or in paired-end mode. The result is FASTQ files containing raw RNA sequencing data.

New samples may be analyzed using one of the methods described, adapting the procedure to the specifics of the study and the available equipment and materials. It is important to consider that cohorts may be prepared in different ways, which affects the stages of sample processing and may be relevant to the comparability of results. The flexibility of these methods allows them to be adapted to various research settings and can help increase the efficiency and accuracy of platelet RNA sequencing.

The data used in the study came from two publicly available sources-GSE183635 [10] (cohort 1) and PRJNA531691 [9] (cohort 2)-as well as from biobanked samples at the Medical University of Gdańsk (cohort 3). Cohort 1 consisted of 2,351 samples from multiple European institutions. It included 390 samples from healthy donors, 144 samples from donors with OC, 1,484 samples from patients with other cancer types (17 cancer types), and 333 samples from donors with non-malignant conditions, e.g., angina, intestinal diseases, hematuria, epilepsy, multiple sclerosis, pancreatic diseases, pulmonary hypertension, as well as donors with cured sarcoma (these samples served as an additional test set). Cohort 2 contained 88 samples collected at different time points from 38 healthy donors. Cohort 3 consisted of 7 samples from healthy donors and 32 samples from donors with OC. The FASTQ files obtained from the sequencer were then processed bioinformatically in accordance with the procedure disclosed in Best M. G. et al., 2019. Reads were mapped to the human reference genome (hg19) using Ensembl gene annotations. Gene coverage analysis was then performed, applying a cutoff of 100,000 reads per sample to improve result reliability. Ultimately, each analyzed sample contained information on RNA expression levels for 5,277 genes measured in platelets.

In developing the model underlying the invention, two different sample sets were combined: collected in Europe cohort 1 and collected in the United States cohort 2. The two cohorts were then normalized together using a variance-stabilizing transformation with the DESeq2 package in R. At the initial stage of the research, a joint normalization of cohorts 1 and 2 was chosen. Data from cohort 3 (samples collected in Gdańsk) were used as an independent test set. They were not combined with cohorts 1 and 2 at this stage in order to increase the credibility of the developed test. Providing independent samples allowed assessment of how the model handles samples to which it had not previously been exposed.

The developed machine-learning models were designed to classify samples as "ovarian cancer" or "healthy". Model training was performed on training data, with classification quality assessed using a validation set. The set sizes are presented in Table 1.

**Table 1. Sample counts from cohort 1 in the training and validation sets (HD - healthy donor, OC - ovarian cancer, M - male, F - female)**

| Tested factor | Compared experiments | Training set [n] | | | | | | Validation set [n] | | | |
|---|---|---|---|---|---|---|---|---|---|---|---|
| | | HD | | OC | | Other cancer types | | HD | | OC | |
| | | M | F | M | F | M | F | M | F | M | F |
| Influence of age as a feature | Samples without age as a separate feature | 158 | 110 | - | 33 | 686 | 575 | - | 71 | - | 102 |
| | Samples with age as a separate feature | 157 | 110 | - | 33 | 682 | 573 | | | | |
| Influence of sex as a feature | Samples from both sexes | 158 | 110 | - | 33 | 686 | 575 | - | 71 | - | 102 |
| | Samples only from women | - | 110 | - | 33 | - | 575 | | | | |
| Impact of including other cancer types in the training set | Samples from all available cancer types | 158 | 110 | - | 33 | 686 | 575 | - | 71 | - | 102 |
| | Only OC samples | 158 | 110 | - | 33 | - | - | | | | |
| The influence of the combination of gender and cancer type on training set | All samples | 158 | 110 | - | 33 | 686 | 575 | - | 71 | - | 102 |
| | Samples only from HD women and OC patients | - | 110 | - | 33 | - | - | | | | |

Based on the logistic regression model, the classification thresholds were appropriately tuned and models were configured for OC screening-tested at a 100% specificity threshold-and for the high-risk group-tested at a 100% sensitivity threshold on the validation set. During training, the impact of various clinical factors on classifier performance metrics was assessed: patient age, sex, and the inclusion of 16 cancer types other than OC in the training dataset. In the method according to the invention, four different comparison modes were applied to determine the best model:
a) training models on samples from both sexes versus training only on samples from women;
b) training models on samples from all available cancer types and from healthy donors versus training only on samples from healthy individuals and OC patients;
c) training models on samples exclusively from healthy women and OC patients versus training models on all available samples;
d) training with age (as a quantitative variable) included as one of the features during training versus training solely on RNA expression profiles;
e) comparing the impact of the type of normalization applied to new samples on classification quality

A simplified workflow of the experiments is shown in Fig. 1. Model hyperparameters were optimized using a grid-search approach implemented via GridSearchCV, focusing on optimizing the area under the ROC (receiver operating characteristic) curve. Model performance was evaluated using a range of metrics, including specificity, sensitivity, accuracy, balanced accuracy, F1 score, and ROC AUC. To estimate 95% confidence intervals (CI) for these metrics, the bootstrap method was used. The implementation of the machine-learning algorithms and analyses was carried out in Python (version 3.10) with the necessary packages, such as scikit-learn (1.2.0), xgboost (1.7.6), imbalanced-learn (0.11.0), numpy (1.22.4), and pandas (1.2.4). These tools provided the framework needed to build, optimize, and evaluate the model, ensuring robustness and reproducibility.

After training the models, classification quality was evaluated on the independent dataset from cohort 3. Each sample was normalized individually together with the data used for model training and validation. This procedure was intended to reduce the influence of the new samples on the normalization. The samples from cohort 3 were divided into subsets of healthy-control samples, cancer samples, and samples with benign conditions. Each subset served as independent test set.

In the first step, models trained on samples collected only from women were compared with models trained on a dataset that included samples from both sexes. The more specialized, albeit smaller, training set enabled the model to capture female-specific gene-expression differences, consistently yielding higher results in both the high-specificity and high-sensitivity models (Fig. 2). Excluding men from the training process increased model accuracy regardless of disease stage. Interestingly, models trained exclusively on samples from women also increased accuracy (from 66.7% to 87.5%) when classifying samples from cohort 2. Similar results were observed in the high-sensitivity model, in which excluding male samples from the training set increased accuracy from 4.2% to 15.9% for early cancer detection. The accuracy of classifying samples from the independent non-malignant control test set decreased slightly (from 80.6% to 76.5%), but increased for the highly sensitive model from 7.6% to 20.6%. A direct comparison of the top 200 features by importance between the model trained on data from both sexes and the model trained solely on female data showed 36.5% overlap, which demonstrates that there are differences in RNA expression between men and women in the tumor profile.

Training high-specificity models exclusively on OC samples, compared with training on data from all available cancer types, showed that while the former approach yielded substantially lower overall sensitivity for OC detection, it achieved better results in early-stage OC detection (Fig. 3). Restricting cancer patients in the training dataset to OC also increased the classification accuracy of non-malignant control samples for the high-specificity model from 80.6% to 91.8%, and of samples from cohort 2 from 66.7% to 89.9%. In the high-sensitivity models, where early-stage specificity was the highest of all experiments, restricting the training data to OC-only samples increased the accuracy for non-malignant controls from 7.6% to 15.8% and for cohort 2 from 4.2% to 6.7%. A direct comparison of the top 200 features by importance between the two models revealed only 13% overlap, suggesting that OC is characterized by a highly distinctive platelet profile.

Combining the conclusions from the previous experiments by restricting the training dataset to samples exclusively from asymptomatic healthy women and patients with OC yielded the most accurate and highly specific early-stage OC detection model (Fig. 4). This model also showed the highest classification accuracy in the non-malignant control group and in cohort 2 (92.4% and 88.3%, respectively). Setting the model to high sensitivity resulted in 36% classification accuracy for cohort 2 and 17.6% for non-malignant samples. Including age as an additional feature in the training set improved the performance of the highly specific and highly sensitive models in detecting OC at late disease stages (Fig. 5).

After the models were trained, they were applied to evaluate samples from the independent cohort 3. When each new sample was normalized separately together with the training cohort, accuracies of 85.7% and 78.13% were observed for asymptomatically healthy controls and for samples from patients with OC, respectively.

### References

1. J. D. Cohen et al., "Detection and localization of surgically resectable cancers with a multi-analyte blood test," Science, vol. 359, no. 6378, pp. 926-930, Feb. 2018, doi: 10.1126/science.aar3247.
2. D. Killock, "CancerSEEK and destroy - a blood test for early cancer detection," Nat Rev Clin Oncol, vol. 15, no. 3, pp. 133-133, Mar. 2018, doi: 10.1038/nrclinonc.2018.21.
3. K.-C. Wong et al., "Early Cancer Detection from Multianalyte Blood Test Results," iScience, vol. 15, pp. 332-341, May 2019, doi: 10.1016/j.isci.2019.04.035.
4. S. Rahaman, X. Li, J. Yu, and K.-C. Wong, "CancerEMC: frontline non-invasive cancer screening from circulating protein biomarkers and mutations in cell-free DNA," Bioinformatics, vol. 37, no. 19, pp. 3319-3327, Jan. 2021, doi: 10.1093/bioinformatics/btab044.
5. C. Sheridan, "Grail to pour $1 billion into blood test to detect early cancer," Nat Biotechnol, vol. 35, no. 2, pp. 101-102, Feb. 2017, doi: 10.1038/nbt0217-101.
6. M. A. Jopek et al., "Improving platelet-RNA-based diagnostics: a comparative analysis of machine learning models for cancer detection and multiclass classification," Molecular Oncology, doi: https://doi.org/10.1002/1878-0261.13689.
7. K. Pastuszak et al., "imPlatelet classifier: image-converted RNA biomarker profiles enable blood-based cancer diagnostics," Mol Oncol, vol. 15, no. 10, pp. 2688-2701, Oct. 2021, doi: 10.1002/1878-0261.13014.
8. Y. Gao et al., "Platelet RNA enables accurate detection of ovarian cancer: an intercontinental, biomarker identification study," Protein & Cell, p. pwac056, Nov. 2022, doi: 10.1093/procel/pwac056.
9. M. T. Rondina et al., "Longitudinal RNA-Seq Analysis of the Repeatability of Gene Expression and Splicing in Human Platelets Identifies a PlateletSELPSplice QTL," Circulation Research, vol. 126, no. 4, pp. 501-516, Feb. 2020, doi: 10.1161/circresaha.119.315215.
10. S. G. J. G. In 't Veld et al., "Detection and localization of early- and late-stage cancers using platelet RNA," Cancer Cell, vol. 40, no. 9, p. 999-1009.e6, Sep. 2022, doi: 10.1016/j.ccell.2022.08.006.
11. M. G. Best, S. G. J. G. In 't Veld, N. Sol, and T. Wurdinger, "RNA sequencing and swarm intelligence-enhanced classification algorithm development for blood-based disease diagnostics using spliced blood platelet RNA," Nature Protocols, vol. 14, no. 4, pp. 1206-1234, Mar. 2019, doi: 10.1038/s41596-019-0139-5.

## Claims

1. A method for analyzing a biological material sample for the detection of ovarian cancer (OC), comprising isolating RNA from platelets, preparing cDNA libraries, fragmenting, molecularly indexing with Illumina barcodes, sequencing, and performing bioinformatic processing, **characterized in that**
- the cDNA libraries, after bioinformatic processing, are mapped to the human reference genome (hg19);
- a cDNA sample with a read count greater than 100,000 per sample is selected;
- the data is normalized using a variance-stabilizing transformation;
- the model training is performed on training data, and assessment of classification quality is performed on the validation set;
- the sample is evaluated by the resulting algorithm, which classifies the sample as "ovarian cancer" or " healthy individual."

2. The method according to claim 1, **characterized in that** each cDNA sample contains information on RNA expression levels for 5,277 genes found in platelets.

3. The method according to claim 1, **characterized in that** the algorithm was trained on samples from two independent cohorts 1 and 2, which were combined and normalized using the DESeq2 package in R.

4. The method according to any one of claims 1 to 3, **characterized in that** the samples from the cohorts training dataset originate from healthy donors and donors with OC.

5. The method according to any one of claims 1 to 4, **characterized in that** the training of the algorithm was limited to samples from asymptomatic healthy women and women with OC.

6. The method according to any one of claims 1 to 5, **characterized in that,** for all cancer stages, the specificity of the method is at least 54.9% for a model with 100% sensitivity, and the sensitivity is at least 74.5% for a model with 100% specificity, when only women are included in the model.

7. The method according to any one of claims 1 to 5, **characterized in that,** for all cancer stages, the specificity of the method is at least 35.2% for a model with 100% sensitivity, and the sensitivity is at least 52.9% for a model with 100% specificity, when only OC are included in the model.

8. The method according to any one of claims 1 to 5, **characterized in that,** for all cancer stages, the specificity of the method is at least 40.8% for a model with 100% sensitivity, and the sensitivity is at least 53.9% for a model with 100% specificity, when only women and OC are included in the model.

9. The method according to any one of claims 1 to 5, **characterized in that,** for all cancer stages, the specificity of the method is at least 59.2% for a model with 100% sensitivity, and the sensitivity is at least 75.5% for a model with 100% specificity, when the platelet RNA profile and donor age are included in the model as a separate feature.
